# EUROPEAN PATENT SPECIFICATION

(11) **EP 3 193 159 B1**
(45) Date of publication and mention of the grant of the patent: **12.09.2018**
(21) Application number: 17151329.4
(22) Date of filing: 13.01.2017
(51) Int. Cl.: G01N 21/3504, G01N 21/39, G01N 33/14

(54) **A PLANT FOR AUTOMATIC FILLING AND/OR PACKAGING OF CLOSED CONTAINERS AND A METHOD FOR MEASURING GAS CONTENT IN CLOSED CONTAINERS**
EINE ANLAGE ZUR AUTOMATISCHEN FÜLLUNG UND / ODER VERPACKUNG VON GESCHLOSSENEN BEHÄLTERN UND EIN VERFAHREN ZUR MESSUNG DES GASINHALTS IN GESCHLOSSENEN BEHÄLTERN
UNE INSTALLATION POUR REMPLISSAGE ET / OU D'EMBALLAGE AUTOMATIQUE DES RÉCIPIENTS FERMÉS ET& xA;UN PROCÉDÉ DE MESURE DU CONTENU DE GAZ DANS DES RÉCIPIENTS FERMÉS

(30) Priority: 13.01.2016 IT UB20169954
(43) Date of publication of application: 19.07.2017
(60) Divisional application: 18185342.5
(73) Proprietor: L PRO S.r.l., 35141 Padova (IT)
(72) Inventor: TONDELLO, GIUSEPPE, 35141 PADOVA (IT)
(74) Representative: Robba, Pierpaolo

(56) References cited:
- EP-A1- 2 372 344
- WO-A1-97/43619
- WO-A1-2008/103837
- CN-B- 103 389 283
- JP-A- 2002 202 247
- US-A- 3 509 996
- US-A1- 2005 084 974
- US-A1- 2015 329 267
- US-B1- 6 694 157
- COCOLA L ET AL: "Validation and calibration of a TDLAS oxygen sensor for in-line measurement on flow-packed products", OPTICAL SENSING II, SPIE, 1000 20TH ST. BELLINGHAM WA 98225-6705 USA, vol. 9855, 12 May 2016 (2016-05-12), pages 98550F-98550F, XP060065766, ISSN: 0277-786X, DOI: 10.1117/12.2228911 ISBN: 978-1-62841-971-9
- D. Masiyano ET AL: "Use of diffuse reflections in tunable diode laser absorption spectroscopy: implications of laser speckle for gas absorption measurements", Applied Physics B: Lasers and Optics, vol. 90, no. 2, 1 February 2008 (2008-02-01), pages 279-288, XP055157825, ISSN: 0946-2171, DOI: 10.1007/s00340-007-2896-z

## Description

The present invention relates to a measuring method of the gas content in closed containers, as well as an automatic filling and/or packaging plant. In particular, the present invention relates to a group and a method for the contactless measurement of the gas contained in closed containers with a high measuring accuracy.

Specifically, the invention relates to a method for measuring the gas content in closed containers, at least partially made of optically transparent material, in particular plastic or glass material, such as for example, but not exclusively, vials or ampoules for powder drugs or liquids, bottles for beverages in general, jars for liquid food or freeze-dried products, and so on.
In container filling and/or packaging plants, an important verification required is checking the gas content inside the container, once filled and closed. For example, many type of containers such as vials and ampoules are sealed and filled with an inert gas and/or maintained at a low internal pressure. For such containers, measuring the residual oxygen content provides an important check, in particular of the container seal. Likewise, in the freeze-dried products, the water vapour relief within the vial is an important check of the lyophilisation process.
To date, numerous invasive measurement techniques are known which thus enable simple sample testing and non-negligible execution times.
For the measurement of the gas content inside containers at least partially made of optically transparent material, in particular plastic material, glass or other similar material, the possibility of using so-called laser spectroscopy measuring instruments is known.
In the present description and in the following claims, the expression "at least partially optically transparent material" means a material with such an absorbance that allows a photodetector to be sensitive to an optical signal transmitted by a light source and having an optical path passing through such a material.

In recent years, laser spectroscopy of TDLAS type (Tuneable Diode Laser Absorption Spectroscopy), which makes use of a compact solid state and robust laser diode, has been widely introduced in industrial applications. In TDLAS, the laser wavelength is tuned in resonance with one or more absorption lines of the gas molecules to be tested, through a linear modulation of the driving current. The signal measured by the detector over time is proportional to the absorption of the sample as a function of the wavelength.

Within this technique, it is envisaged that a laser beam with a wavelength specially tuned passes through the path in which the gas is located. After such a pass, the laser beam is detected by a photodiode or other suitable detector. It is known to use VCSEL (Vertical Cavity Surface Emitting Lasers) or DFB (Distributed FeedBack Lasers) as tuneable lasers for industrial applications.

In exemplary terms, a widespread application that makes use of this measurement technique is the in-line check of the CO₂ contents and pressure in wine and soft drink bottles, as described for example in the international patent application no. WO 2008/53507. According to this measurement technique, a beam emitted by a tuneable laser diode operating in the near-infrared and synchronized in resonance with some absorption lines of the carbon dioxide molecule is sent through the head space of the container. In output, the laser beam is changed by the absorption undergone by passing through the gas. This output laser beam is analysed in order to determine the intensity and shape of the absorbed lines and, from these quantities, derive the concentration and pressure of CO₂ gas inside the container.

In the present description and following claims, the term "head space" means the area enclosed in the closed container in which the filling material (such as liquid or solid) is not present. In case of a container arranged with closure upwards, the head space is the area between the free surface of the material in the container (such as liquid or powders) and the closure (such as the cap) of the container. Alternatively, if the container is upside down, the head space indicates the area between the free surface of the material in the container and the bottom of the container.

A further exemplary application that makes use of laser spectroscopy is the verification of the residual oxygen and/or water vapour content in vials and/or ampoules as described in U.S. patent 6.639 .678. A laser beam tuned to the absorption lines of oxygen or water vapour is sent through the head space of a vial and is detected by a photodiode. This specific case uses a variant of the TDLAS technique, specifically it uses the WMS (Wavelength Modulation Spectroscopy) method, which involves superimposing the linear scanning of the wavelength with a sinusoidal modulation at a suitable higher frequency and, simultaneously, the demodulation of the received signal. Information on absorption is contained in higher-order harmonics of the modulation frequency, typically the first and second ones. In this way, higher sensitivity can be achieved, making this method especially appropriate in the case of low absorbance of the sample to be tested, as in the specific case of oxygen detection.

US 2005/084974 A1 discloses sensing devices using laser spectroscopy in headspace sensing and analysis of sealed optically-transparent containers that are moved along a conveyor. Tunable diode laser absorption spectroscopy (TDLAS) is used which is well-suited to monitoring gases in small volume containers for manufacturing process and quality control applications.

In general terms, absorption spectroscopy is based on Beer-Lambert law according to which, the transmission of light through a sample obeys the law I = Io exp[-σ(λ)NL] (where I is the intensity of the light after the absorption path, Io is the intensity of the laser beam emitted, σ(λ) is the row section depending on the molecule and on the wavelength, N is the concentration of absorbing molecules and L is the optical path within the sample to be analysed).
Absorbance, i.e. the amount of light absorbed with respect to the laser light emitted, is defined as follows: (Io -I) /Io.
The Applicant noted that in the detection of oxygen in vials and ampoules, absorbance is greatly reduced. This is both because the absorption section σ(λ) of the oxygen row is inherently very small, oxygen molecules being homonuclear, and because the optical path inside the vial and the residual oxygen concentration are both small. By way of example, in the case of vials of lyophilized product, it is usual to have optical paths of about 2 cm and a residual oxygen concentration equal to 1%. Under such conditions, absorbance values are obtained which are in the order of magnitude of 10⁻⁵ which are hardly detectable with repeatability and reliability as required in an industrial context. The Applicant further noted that, among the factors that limit the accuracy and precision of measurements in TDLAS-WMS applications are also the random interference between highly coherent laser beams that are diffused or reflected by various surfaces that the laser beam crosses on its way from the diode laser to the detector. Such disturbances, known etalon effect, lead to irregular oscillations of the signal received by the detector and are often a limiting factor of the sensitivity and reproducibility of the TDLAS technique used on the field. In order to reduce the etalon effect, it is known to introduce random vibrations of the optical system, namely of all the surfaces crossed by the laser beam, but this often is not applicable in the industry.
The Applicant also noted that a further factor of interference in practical application is the type of glass of vials, which is not always of suitable quality, for example subsequent to an uneven wall thickness. Such walls influence the measurement since their crossing by the laser beam may cause a change in the trajectory and therefore in the stream received by the detector, as well as introduce a random scattering of light.
Changes in trajectory and consequent laser beam misalignments with respect to the detector are also introduced by any mechanical vibration of the feeding system of containers in industrial applications, as well as by temperature changes of the environment in which they operate.

The problem underlying the present invention therefore is to measure gas content in closed containers to provide accurate, repeatable and reliable, measurements even under reduced optical path conditions or small residual amount of the gas to be measured.

Within the scope of this problem, an object of the present invention is to measure the gas content in closed containers in an industrial application and at the same time to avoid an etalon effect, thus providing an increased measurement accuracy.

In particular, a further object of the present invention is to measure the gas content in closed containers without being affected either by any laser beam misalignments with respect to the detector, or by the optical qualities of the walls of the containers.

The Applicant has perceived that, through the use of at least one diffusive wall portion suitably arranged with respect to the laser source and the detector, the paths that the incoming light crosses through the head space before reaching the detector may be multiplied. In this way, the light reaching the detector contains a lot of information in relation to the gas content inside the container, having absorbance values sufficient to allow a reliable and repeatable measurement of the gas content even in case of measurement of the oxygen content.
The Applicant has therefore identified the optimal mutual arrangement to achieve a multiplication of the paths crossed by the laser light and therefore a significant increases of the equivalent path, reproducing an effect comparable to that provided by an integrating sphere. This allows obtaining accurate, repeatable and reliable measurements even under conditions of reduced actual optical path or minimal residual amount of the gas to be measured.
The Applicant also noted that an increase in the accuracy and reliability of the measurements is confirmed independently of the geometry of the surface portion with diffusive optical properties, always ensuring a considerable increase of the equivalent path and therefore a more robust measurement, insensitive to the irregularities of the containers with respect to the prior art measuring groups. Moreover, the measuring group allows achieving a high degree of sturdiness also with respect to the alignment of the laser beam emitted by the source, thereby allowing also the use of non-collimated sources, such as a diode laser or an optical fibre.
The Applicant has further found that the particular elements that make up the measuring group and their respective mutual arrangement are able to substantially eliminate the etalon effect, offering particularly accurate measurements. The measuring group therefore proves particularly suitable for use in industrial applications.

This is also confirmed in the case of a container with highly irregular walls, such as happens in glass vials obtained by moulding. The Applicant has in fact found that the measurements obtained by means of the measuring group are not affected by such irregularities.
According to a second aspect thereof, the invention relates to a method for measuring the gas content in closed containers according to claim 5 as appended. Advantageously, the method for measuring the gas content in closed containers according to the invention achieves the technical effects described above with reference to the measuring group of the gas content in closed containers.

According to a first aspect thereof, the invention relates to a plant for automatic filling and/or packaging of closed containers according to appended claim 1. Advantageously, the automatic filling and/or packaging plant according to the invention achieves the technical effects described above in relation to the measuring group of the pressure in closed containers.
The present invention may have at least one of the following preferred features; the latter may in particular be combined with each other as desired to meet specific application requirements.
Preferably, the detector is positioned near the laser source.
More preferably, the detector and the laser source have the same orientation.
The detector positioned near the laser source and, in particular, the same orientation ensure that a laser beam is detected which has carried out at least two passes through the container also in case of containers with non-optimal optical features, such as amber containers.
Preferably, the delimiting surface of the at least one housing seat is the inner surface of a box-shaped housing body of a container.
More preferably, the portion with high diffusion coefficient coincides with the entire inner surface of the box-shaped body or with part thereof.
More preferably, the box-shaped body for housing a container has a cylindrical, parallelepiped or spherical configuration.
More preferably, the box-shaped body for housing a container has an internal configuration substantially matching the configuration of the container.

More preferably, the box-shaped body for housing a container comprises, in its own wall, at least two openings, a first opening having dimensions sufficient to allow the entry of a laser light generated by the at least one laser source and a second opening being suitable for allowing the laser light diffused inside the box-shaped body to exit.

Even more preferably, at least one laser source being arranged at the first opening and the detector being positioned at the second opening.

Alternatively, the delimiting surface of the at least one housing seat is a wall positioned facing the at least one laser source.

Preferably, the wall facing at least one laser source is arched.

Preferably, the at least one laser source is of the type suitable for emitting a collimated beam or a diverging beam, preferably a laser diode or an optical fibre.

Preferably, the measuring group further comprises flushing means for introducing a different gas from the one under examination into the housing seat.

This advantageously allows reducing or eliminating the contribution of molecular absorption given by the gas under examination present outside the container, such as in the case of oxygen measurement, and hence the influence on the overall measurement. In the case of oxygen measurement, it is advantageous to flush nitrogen into the housing seat in order to eliminate the oxygen present in the seat outside the container.

Preferably, the conveyor is of the carousel type.

Preferably, each compartment of the plurality of compartments housing the containers is defined by a substantially half-cylindrical wall.

Preferably, the laser source and the detector of the measuring group are comprised in a measuring head and face two respective openings towards an open box-shaped body.

More preferably, the open box-shaped body extends in width so as to wrap around at least part of the compartments adjacent to the compartment of the container positioned at the at least one measuring group.

More preferably, the open box-shaped body comprises a second portion made of a high diffusion coefficient material.

Preferably, the high diffusion coefficient portions are portions painted of an opaque white colour or made of white material or other material with a high diffusion coefficient.
Preferably, the conveyor is controllable according to a continuous or jigging motion with a stop in a container at the measuring group for the duration of the measurement. Further features and advantages of the present invention will appear more clearly from the following detailed description of some preferred embodiments thereof, made with reference to the accompanying drawings.
The different features in the single configurations may be combined with one another as desired according to the description above, to make use of the advantages resulting in a specific way from a particular combination.
In such drawings,
- figure 1 is a schematic side elevational view of a first example of the measuring group of the gas content in a closed container according to the present disclosure, with a container inserted therein;
- figure 1a is a sectional view along line A-A of the measuring group in figure 1;
- figure 1b is a sectional view along line B-B of the measuring group in figure 1;
- figure 2 is a schematic side elevational view of a second example of the measuring group of the gas content in a closed container according to the present disclosure, with a container inserted therein;
- figure 2a is a sectional view along line A'-A' of the measuring group in figure 2;
- figure 2b is a sectional view along line B'-B' of the measuring group in figure 2;
- figure 3 is a schematic side elevational view of a third example of the measuring group of the gas content in a closed container according to the present disclosure, with a container inserted therein;
- figure 3a is a sectional view along line A"-A" of the measuring group in figure 3;
- figure 3b is a sectional view along line B"-B" of the measuring group in figure 3;
- figure 4a is a schematic plan view of a carousel of a filling and/or packaging plant comprising a fourth embodiment of the measuring group of the gas content in a closed container according to the present invention;
- figure 4b is a sectional view along plane C-C of the carousel in figure 4a;
- figure 4c is an enlarged detail of figure 4b which shows the laser source and the photodetector of the measuring group.

In the following description, identical reference numerals are used for the illustration of the figures to indicate construction elements having the same function. Moreover, for clarity of illustration, some numerical references are not repeated in all the figures.

With reference to the figures, some embodiments are shown of a measuring group of the gas content in closed containers, globally indicated with reference numerals 10, 10', 10".

In the present description and in the following claims, it is assumed that the closed containers 30, 30", 130 subjected to check are made of an optically transparent material at least at a portion of the head space 31, 131 thereof.

The measuring groups 10, 10', 10", 100 shown in the figures all comprise a laser source 22, 122 for emitting a laser beam 20, 120 at a wavelength tuneable with an absorption wavelength of a gas contained in the head space 31 of the closed container 30, 30', 30", 130. The laser source 22, 122 is facing a surface 17, 18, 117 comprising at least one portion 16, 16', 16", 116 with a high diffusion coefficient or also diffusive portion. A detector 21,121 is further provided, such as a photodiode, also at least partially facing the diffusive portion 16,16',16",116.

A housing seat 14, 114 is defined in the space defined between the laser source 22, 122, the diffusive portion 16, 16', 16", 116 and detector 21,121 which is suitable for housing at least a part of a closed container 30, 30', 30", 130 and in particular, of a head space 31, 131 of the container or, in particular, of the portion of head space 31, 131 made of optically transparent material.

The laser source 22, 122 is positioned in such a way as to direct the laser beam towards the diffusive portion 16, 16', 16", 116 passing through the housing seat 14, 114 or the optically transparent portion of the head space 31, 131 of a container 30, 30', 30", 130 arranged in such a seat.

Detector 21, 121 is oriented in such a way as to receive a plurality of laser beams diffused by portion 16, 16', 16", 116. Based on the beams received, detector 21.121 is adapted to provide an output data representative of the absorption spectrum of the gas contained in the head space 31, 131 of container 30, 30', 30", 130 housed into seat 14, 114.

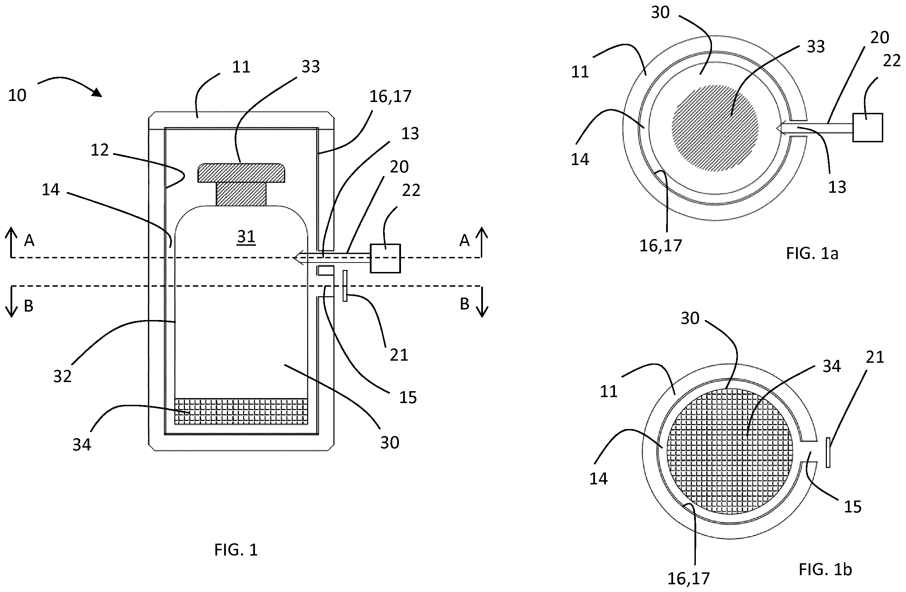

With specific reference to figures 1, 1a and 1b, surface 17 comprising at least one diffusive portion 16 is the inner surface of a cylindrical box-shaped body 11, inside of which the housing seat 14 of a container 30 is defined. In the specific example discussed, the diffusive portion 16 coincides with the entire inner surface 17 of the box-shaped body 11.
Seat 14 defined by the box-shaped body 11 is shaped in such a way as to house the entire container 30 and therefore, in the specific case, not only the head space 31 of the same 30.
Two openings 13, 15 having small dimensions are formed in wall 12 of the box-shaped cylindrical body 11. In particular, a first opening 13 has dimensions sufficient to allow the entry of a laser light 20 generated by the laser source 22 and is arranged at the head space 31 of container 30. A second opening 15 is such as to allow the output passage of the laser light diffused inside the box-shaped body 11. Detector 21 is positioned at the second opening 15 in order to receive the output light.

The first example is particularly suitable for measuring the gas content inside a container 30 which houses a freeze-dried or solid product 34. Such containers are typically characterized by a large head space 31 delimited by walls 32 of optically transparent material and usually with uniform thickness, thus of good optical quality. Under such conditions, the laser light 20 entering through the first opening 13 crosses the head space 31, reaching the inner wall 17 of the box-shaped body 11. Due to the diffusive optical properties of the entire inner wall 17, the laser light 20 is diffused in all directions, again crossing the head space 31 and impinging other portions of the inner wall 17 of the box-shaped body 11, whereby it is diffused again. Due to the large optically transparent portion of container 30, substantially the entire diffusive portion 16 of the inner wall 17 of the box-shaped body 11 is impinged by the diffused light, thus contributing to diffused the laser light 20 again. Only the diffused laser light 20 impinging any opaque parts of container 30, such as closure 33 of container 30 or product 34 included in container 30, is not diffused again.
Detector 21 positioned at the second opening 15, therefore receives a contribution of light, of which part has passed several times through the head space 31, thus containing a large information contribution as regards the gas content inside container 30.

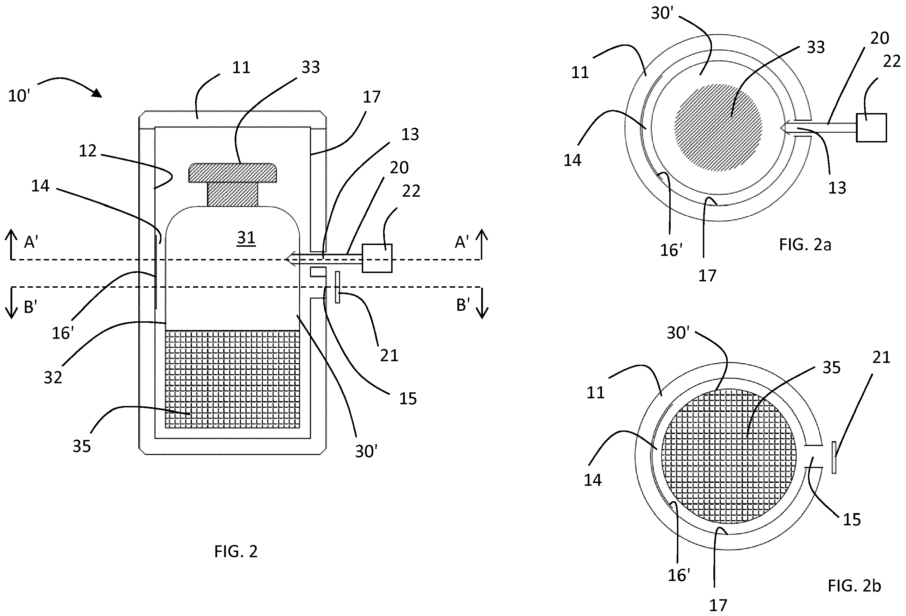

With reference to the second embodiment shown in figures 2, 2a and 2b, the at least partially diffusive portion 16' is a part of the inner surface 17 of a cylindrical box-shaped body 11, inside of which the housing seat 14 of a container 30 is defined.

In particular, the diffusive surface portion 16' is arranged at the height where the head space 31 of container 30' is positioned and is facing the first opening 13.

The second embodiment is particularly suitable for measuring the gas content inside a container 30 which houses a liquid product 35, such containers 30' being generally filled by a non-negligible volume.

Under such conditions, the laser light 20 entering through the first opening 13 crosses the head space 31, reaching the diffusive portion 16' of the inner wall 17 of the box-shaped body 11. Due to the optical properties of the diffusive portion 16', the laser light 20 is diffused in all directions, again crossing the head space 31 and partially also impinging the diffusive portion 16' again, whereby it is diffused again. The diffused laser light 20 impinging the non-diffusive inner wall 17 portions and the opaque parts of container 30', such as closure 33 of container 30' or liquid 35 included in container 30', is not diffused again or is only partially diffused again.

Anyway, also in this case detector 21 positioned at the second opening 15 receives a contribution of light that has passed at least twice through the head space 31, thus containing an increased information contribution as regards the gas content inside container 30', with respect to a single pass through such a space 31.

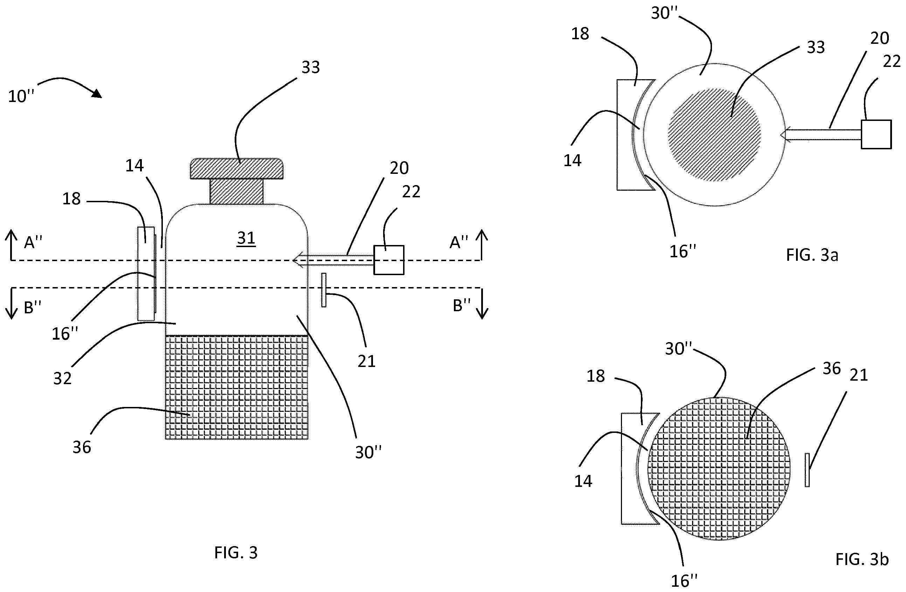

With reference to the third embodiment shown in figures 3, 3a and 3b, the at least partially diffusive portion 16" is an arched wall 18, in particular having a configuration matching at least part of the outer surface of a closed container 30". The arched wall 18 is positioned facing the laser source 22 and detector 21. The housing seat 14 suitable for housing the head space 31 of container 30" is defined between the laser source 22, the diffusive portion 16" and detector 21.

The third embodiment is also particularly suitable for measuring the gas content inside a container 30" which comprises an amount of product 36 sufficient for filling most of container 30", where the arched wall 18 is placed at a higher height than that of the free surface of product 36 contained in container 30" when this is housed in seat 14.

Under such conditions, the laser light 20 emitted by source 22 crosses the head space 31, reaching the diffusive portion 16" of the arched wall 18. Due to the optical properties of the diffusive portion 16", the laser light 20 is diffused in all directions, again crossing the head space 31 and partially also impinging the diffusive portion 16" again, whereby it is diffused again.

Anyway, also in this case detector 21 positioned at the second opening 15 receives a contribution of light that has passed at least twice through the head space 31, thus containing an increased information contribution as regards the gas content inside container 30", with respect to a single pass through such a space 31.

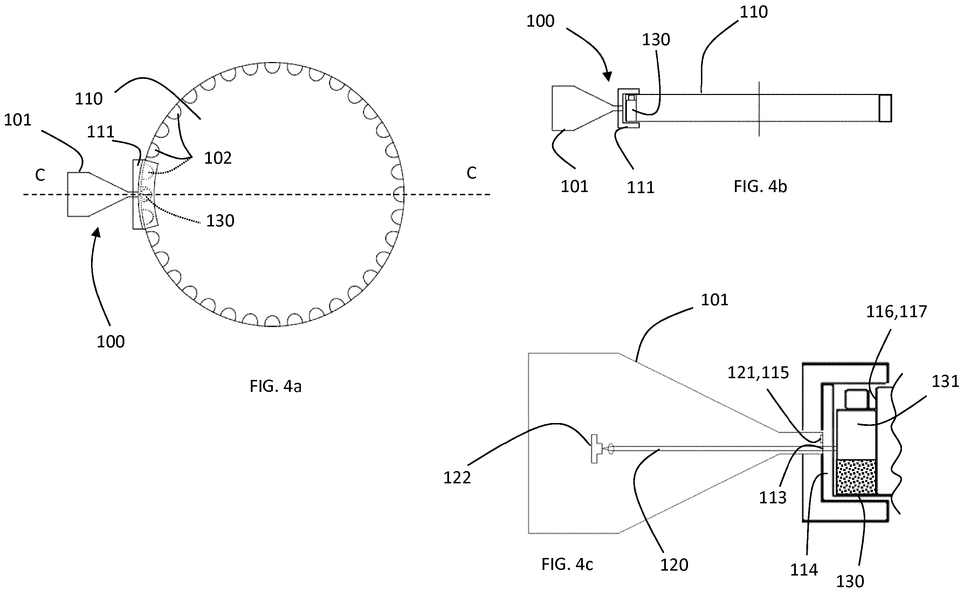

With reference to the fourth embodiment shown in figures 4a, 4b and 4c, the measuring group 100 of the gas content in a closed container is associated with a carousel conveyor 110 for feeding containers 130 inside an automatic filling and/or packaging plant (not shown).

Such a measuring group 100 comprises a measuring head 101 within which both a laser source 122 and a detector 121 are arranged. The laser source 122 and detector 121 face from two respective openings 113,115 towards an open box-shaped body 111 which defines part of a housing seat 114 of a container 130 and in particular, of the respective head space 131.

The carousel conveyor 110 comprises a plurality of compartments 102 for housing containers 131, each defined by a substantially semi-cylindrical wall 117, comprising at least one portion 116 with a high diffusion coefficient. Such compartments 102 contribute, with the open box-shaped body 111 of the measuring group 100, to form the housing seat 114 of container 130.

The open box-shaped body 111 extends in width so as to preferably wrap around at least part of compartments 102 adjacent to the carousel conveyor 110 with respect to compartment 102 of container 130 subject to measurement. The open box-shaped body 111 also advantageously comprises at least one portion made of a material with a high diffusion coefficient, such as Teflon or other white or white coloured material.

As the rotation of the carousel conveyor 110 progresses, each container 130 carried by the same passes in front of the measuring head 101, housed in seat 114 defined between the open box-shaped body 111 and compartment 102 of the carousel conveyor 110 in which container 130 is carried. In this way, light 120 emitted by the laser source 122 is diffused by the diffusive portions 116 present in compartment 114 and preferably, also in the open box-shaped body 111, returning to detector 121 after passing several times through the head space 131 of container 130.
The operation of the measuring group 10 of pressure in closed containers is as follows. The measuring group is first calibrated in order to determine the multiplication factor of the equivalent path provided by the specific measuring group when operated to take the measurement on a specific container.
To this end, measurements are carried out on sample containers substantially identical to containers 30, 130 to be subjected to measurement in terms of filling and optical quality of the walls. Thereafter, the corresponding gas contents of such sample containers are measured by means of conventional measurement methods, possibly of destructive type. In this way, it is possible to quantify the multiplicative effect induced by the measuring group on the equivalent path, thus determining a corresponding calibration data of the measuring group 10, 10', 10", 100.
Thereafter, containers 30, 130 subject to measurement are carried one at a time at the housing seat 14, 114 defined between the laser source 22, 122, the diffusive portion 16, 16', 16", 116 and detector 21, 121.
The laser source 20, 120 then emits a laser beam directed towards the diffusive portion 16, 16', 16", 116, passing through the housing seat 14, 114. In particular, the laser beam is emitted towards the head space 31, 131 of container 30, 130 located in the housing seat 14, 114, and precisely at the portion of container 30, 130 made of optically transparent material.
Detector 21, 121 detects the laser beams diffused by the diffusive portion 16, 16', 16", 116 and attenuated following the absorption that has taken place at the head space 31, 131 of container 30, 130 and for each laser beam detected, it provides output data representative of the absorption spectrum of the gas subject to measurement present in the head space 31, 131 as a function of the calibration data initially determined.

The features of the method of measuring the gas content in closed containers as well as of the relative filling and/or packaging plant object of the present invention are clear from the above description, as are its advantages.
Additional variations of the embodiments described above are possible without departing from the teaching of the invention.

Finally, it is clear that several changes and variations may be made to the method of measuring the gas content in closed containers and to a relative filling and/or packaging plant thus conceived, all falling within the invention; moreover, all details can be replaced with technically equivalent elements. In the practice, the materials used as well as the sizes, can be whatever, according to the technical requirements.

## Claims

1. A plant for automatic filling and/or packaging of closed containers (130), the containers being made from optically transparent material at least at a portion of a head space (131) thereof, the plant comprising:
at least one conveyor (110) adapted to advance a plurality of containers (130) along an advancing path, the conveyor comprising a plurality of partially open compartments (102) for housing containers (130) each defined by at least one wall (117),
at least one measuring group (100) for measuring the gas content in the closed containers (130) made from optically transparent material at least at a portion of a head space (131) thereof, the group comprising at least one laser source (122) for emitting a laser beam (120) at a wavelength tuneable with an absorption wavelength of a gas contained in the head space (131) of the closed container (130), and at least one detector (121) adapted to detect at least one portion of the laser beam (120) emitted by the laser source (122) and for providing, in output, data representative of an absorption spectrum of the gas;
wherein the at least one conveyor (110) is adapted to transport each container to the at least one measuring group (100);
**characterised in that** the wall (117) of each partially open compartment (102) for housing a container (130) comprises at least one portion (116) with high optical scattering coefficient, and **in that**, when a container (130) is at the at least one measuring group (100), the portion (116) of the compartment wall with high optical scattering coefficient
faces the at least one laser source (122) and the at least one detector (121) is positioned so as to detect at least one portion of the laser beam (120) emitted by the laser source (122) which has been scattered at least once by the wall portion (116) with high optical scattering coefficient.

2. A plant for automatic filling and/or packaging according to claim 1, wherein the laser source (122) and the detector (121) of the measuring group (100) are comprised in a measuring head (101) and face two respective openings (113,115) towards an open box-shaped body (111).

3. A plant for automatic filling and/or packaging according to claim 2, wherein the open box-shaped body (111) extends in width so as to wrap around at least part of the compartments (102) adjacent to the compartment (102) of the container (130) positioned at the at least one measuring group (100).

4. A plant for automatic filling and/or packaging according to claim 2 or 3, wherein the open box-shaped body (111) comprises a second portion made from material with high optical scattering coefficient.

5. Method for measuring the gas content in closed containers (30,30',30",130) made from optically transparent material at least at a portion of a head space (31,131) the method comprising the steps consisting of:
- housing the at least one portion of a head space (31,131) of a container (30,30',30",130) of such closed containers in a housing seat (14,114) defining a space delimited at least by a surface (17,18,118) of the seat, the surface comprising at least one portion (16,16',16",116) with high optical scattering coefficient, the portion with high scattering coefficient (16,16',16",116) facing towards a laser source;
- emitting a laser beam (20,120) at a wavelength tuneable with an absorption wavelength of a gas contained in the head space (31,131) of the closed container (30,30',30",130) towards the housing seat (14,114);
- detecting at least one portion of the laser beam (20,120) that has passed through space defined by the housing seat (14,114) that has been scattered by the surface portion (16,16',16",116) with high optical scattering coefficient and that has passed a plurality of times through the space and the headspace in the container, and providing, in output, data representative of an absorption spectrum of the gas.

## Patentansprüche

1. Anlage zur automatischen Füllung und/oder Verpackung von geschlossenen Behältern (130), wobei die Behälter zumindest in einem Bereich eines Kopfraums (131) aus einem optisch transparenten Material sind, wobei die Anlage aufweist:
wenigstens einen Förderer (110), der dazu vorgesehen ist, mehrere Behälter (130) entlang eines Vorschubwegs zu bewegen, wobei der Förderer mehrere teilweise offene Fächer (102) zum Aufnehmen der Behälter (130) aufweist, die jeweils durch wenigstens eine Wand (117) gebildet sind,
wenigstens einer Messgruppe (100) zum Messen des Gasgehalts in den geschlossenen Behältern (130), die zumindest in einem Bereich eines Kopfraums (131) aus optisch transparentem Material sind, wobei die Gruppe wenigstens eine Laserquelle (122) zum Aussenden eines Laserstrahls (120) bei einer Wellenlänge aufweist, die auf eine Absorptionswellenlänge eines in dem Kopfraum (131) des geschlossenen Behälters (130) enthaltenen Gases abstimmbar ist, und wenigstens einen Detektor (121), der dazu vorgesehen ist, wenigstens einen Teil des von der Laserquelle (122) ausgesandten Laserstrahls (120) zu detektieren und als Ergebnis Daten zu liefern, die für ein Absorptionsspektrum des Gases repräsentativ sind;
wobei der wenigstens eine Förderer (110) dazu vorgesehen ist, jeden Behälter zu der wenigstens einen Messgruppe (100) zu transportieren;
**dadurch gekennzeichnet, dass** die Wand (117) von jedem teilweise offenen Fach (102) zum Aufnehmen eines Behälters (130) wenigstens einen Abschnitt (116) mit einem hohen optischen Streukoeffizienten aufweist und, dass, wenn ein Behälter (130) bei der wenigstens einen Messgruppe (100) ist, der Abschnitt (116) der Wand des Fachs mit dem hohen optischen Streukoeffizienten der wenigstens einen Laserquelle (122) zugewandt ist und der wenigstens eine Detektor (121) so angeordnet ist, dass wenigstens ein Abschnitt des von der Laserquelle (122) ausgesandten Laserstrahls (120) detektiert wird, der von dem Wandabschnitt (116) mit dem hohen optischen Streukoeffizienten wenigstens einmal gestreut wurde.

2. Anlage zur automatischen Füllung und/oder Verpackung nach Anspruch 1, wobei die Laserquelle (122) und der Detektor (121) der Messgruppe (100) einen Messkopf (101) aufweisen und zwei entsprechenden Öffnungen (113, 115) eines offenen kastenförmigen Körpers (111) zugewandt sind.

3. Anlage zur automatischen Füllung und/oder Verpackung nach Anspruch 2, wobei der offene kastenförmige Körper (111) sich in der Breite so erstreckt, dass er wenigstens einen Teil der Fächer (102) umgibt, die an das Fach (102) des Behälters (130) angrenzen, der an wenigstens einen Messgruppe (100) positioniert ist.

4. Anlage zur automatischen Füllung und/oder Verpackung nach Anspruch 2 oder 3, wobei der offene kastenförmige Körper (111) einen zweiten Abschnitt aufweist, der aus Material mit einem hohen optischen Streukoeffizienten hergestellt ist.

5. Verfahren zum Messen des Gasgehalts in geschlossenen Behältern (30, 30', 30", 130), die zumindest in einem Bereich eines Kopfraums (31, 131) aus optisch transparentem Material sind, wobei das Verfahren die folgenden Schritte aufweist:
- Aufnehmen des wenigstens einen Bereichs des Kopfraumes (31,131) eines Behälters (30, 30', 30", 130) eines solchen verschlossenen Behälters in einem Aufnahmesitz (14, 114), der einen Raum definiert, der von wenigstens einer Oberfläche (17, 18 , 118) des Sitzes begrenzt ist, wobei die Oberfläche wenigstens einen Bereich (16, 16', 16", 116) mit einem hohen optischen Streukoeffizienten aufweist, wobei der Bereich mit dem hohen Streukoeffizienten (16, 16', 16", 116) einer Laserquelle zugewandt ist;
- Aussenden eines Laserstrahls (20, 120) mit einer Wellenlänge, die auf eine Absorptionswellenlänge eines in dem Kopfraum (31, 131) des geschlossenen Behälters (30, 30', 30", 130) enthaltenen Gases abstimmbar ist, zu dem Aufnahmesitz (14, 114);
- Detektieren von wenigstens einem Teil des Laserstrahls (20, 120), der den durch den Aufnahmesitz (14, 114) definierten Raum durchlaufen hat und von dem Oberflächenbereich (16, 16', 16", 116) mit hohem optischen Streuquerschnitt gestreut wurde und mehrmals durch den Raum und den Kopfraum in dem Behälter durchlaufen hat, und als Ergebnis Daten liefert, die für ein Absorptionsspektrum des Gases repräsentativ sind.

## Revendications

1. Installation de remplissage et/ou d'emballage automatique de contenants fermés (130), les contenants étant réalisés en un matériau optiquement transparent au moins au niveau d'une portion d'un espace libre (131) de ceux-ci, l'installation comprenant :
au moins un convoyeur (110) adapté pour faire avancer une pluralité de contenants (130) le long d'un chemin d'avancement, le convoyeur comprenant une pluralité de compartiments partiellement ouverts (102) pour loger des contenants (130) définis chacun par au moins une paroi (117),
au moins un groupe de mesure (100) pour mesurer la teneur en gaz dans les contenants fermés (130) réalisés en un matériau optiquement transparent au moins au niveau d'une portion d'un espace libre (131) de ceux-ci, le groupe comprenant au moins une source laser (122) pour émettre un faisceau laser (120) à une longueur d'onde accordable avec une longueur d'onde d'absorption d'un gaz contenu dans l'espace libre (131) du contenant fermé (130), et au moins un détecteur (121) adapté pour détecter au moins une portion du faisceau laser (120) émis par la source laser (122) et pour fournir, en sortie, des données représentatives d'un spectre d'absorption du gaz ;
dans laquelle l'au moins un convoyeur (110) est adapté pour transporter chaque contenant vers l'au moins un groupe de mesure (100) ;
**caractérisée en ce que** la paroi (117) de chaque compartiment partiellement ouvert (102) pour loger un contenant (130) comprend au moins une portion (116) avec un coefficient de dispersion optique élevé, et **en ce que**, lorsqu'un contenant (130) est au niveau de l'au moins un groupe de mesure (100), la portion (116) de la paroi de compartiment avec un coefficient de dispersion optique élevé est en regard de l'au moins une source laser (122) et l'au moins un détecteur (121) est positionné de façon à détecter au moins une portion du faisceau laser (120) émis par la source laser (122) qui a été dispersé au moins une fois par la portion de paroi (116) avec un coefficient de dispersion optique élevé.

2. Installation de remplissage et/ou d'emballage automatique selon la revendication 1, dans laquelle la source laser (122) et le détecteur (121) du groupe de mesure (100) sont compris dans une tête de mesure (101) et sont en regard de deux ouvertures (113, 115) respectives vers un corps en forme de boîte ouverte (111).

3. Installation de remplissage et/ou d'emballage automatique selon la revendication 2, dans laquelle le corps en forme de boîte ouverte (111) s'étend en largeur de façon à s'enrouler autour d'au moins une partie des compartiments (102) adjacents au compartiment (102) du contenant (130) positionné au niveau de l'au moins un groupe de mesure (100).

4. Installation de remplissage et/ou d'emballage automatique selon la revendication 2 ou 3, dans laquelle le corps en forme de boîte ouverte (111) comprend une seconde portion réalisée en un matériau avec un coefficient de dispersion optique élevé.

5. Procédé de mesure de la teneur en gaz dans des contenants fermés (30, 30', 30", 130) réalisés en un matériau optiquement transparent au moins au niveau d'une portion d'un espace libre (31, 131), le procédé comprenant les étapes consistant à :
- loger l'au moins une portion d'un espace libre (31, 131) d'un contenant (30, 30', 30", 130) de ces contenants fermés dans un siège de logement (14, 114) définissant un espace délimité au moins par une surface (17, 18, 118) du siège, la surface comprenant au moins une portion (16, 16', 16", 116) avec un coefficient de dispersion optique élevé, la portion avec un coefficient de dispersion élevé (16, 16', 16", 116) étant en regard d'une source laser ;
- émettre un faisceau laser (20, 120) à une longueur d'onde accordable avec une longueur d'onde d'absorption d'un gaz contenu dans l'espace libre (31, 131) du contenant fermé (30, 30', 30", 130) vers le siège de logement (14, 114) ;
- détecter au moins une portion du faisceau laser (20, 120) qui a traversé l'espace défini par le siège de logement (14, 114), qui a été dispersé par la portion de surface (16, 16', 16", 116) avec un coefficient de dispersion optique élevé et qui a traversé une pluralité de fois l'espace et l'espace libre du contenant, et fournir, en sortie, des données représentatives d'un spectre d'absorption du gaz.
